(19) Europäisches Patentamt
European Patent Office
Office européen des brevets

(11) Publication number: **0 221 511 B1**

(12) **EUROPEAN PATENT SPECIFICATION**

(45) Date of publication of patent specification: **02.01.92**

(51) Int. Cl.[5]: **C07D 295/12**, A61K 31/535

(21) Application number: **86115050.6**

(22) Date of filing: **29.10.86**

(54) **2-aminosulfonyl-6-nitrobenzoic esters or amides, process for their preparation and pharmaceutical compositions containing them.**

(30) Priority: **06.11.85 US 795565**

(43) Date of publication of application:
**13.05.87 Bulletin 87/20**

(45) Publication of the grant of the patent:
**02.01.92 Bulletin 92/01**

(84) Designated Contracting States:
**CH DE FR GB IT LI NL**

(56) References cited:
**EP-A- 0 021 592**
**EP-A- 0 197 386**
**US-A- 4 603 133**

(73) Proprietor: **MERCK & CO. INC.**
**126, East Lincoln Avenue P.O. Box 2000**
**Rahway New Jersey 07065-0900(US)**

(72) Inventor: **Engelhardt, Edward L.**
**904 Plymouth Road**
**Gwynedd Valley, PA 19437(US)**
Inventor: **Saari, Walfred S.**
**1740 Wagon Wheel Lane**
**Lansdale, PA 19446(US)**

(74) Representative: **Blum, Rudolf Emil Ernst et al**
**c/o E. Blum & Co Patentanwälte Vorderberg 11**
**CH-8044 Zürich(CH)**

**EP 0 221 511 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

This invention relates to esters, amides and N-substituted amides of 2-[N-(morpholinoalkyl)-aminosulfonyl]-6-nitrobenzoic acids and salts of said compounds. The corresponding compounds are esters or amides of the benzoic acid which are substituted in the o-positions to the ester group, respectively acid amide group with a nitro group as well as an amino sulfonyl group in which there is bonded to the nitrogen atom of the amino sulfonyl group an alkyl chain comprising 1, 2 or 3 carbon atoms which is substituted in the $\omega$-position with the nitrogen atom of a morpholino group. The corresponding esters or amides of the benzoic acid are useful as sensitizers of hypoxic tumor cells to therapeutic radiation.

The invention furthermore concerns pharmaceutical compositions for enhancing the effect of a therapeutic radiation of hypotoxic tumor cells, which compositions contain as pharmaceutically active ingredient the above stated esters or amides of the benzoic acid which are substituted in both ortho-positions of the benzene nucleus. The corresponding pharmaceutical compositions are administered to patients in need of a therapeutic radiation treatment of hypoxic tumor cells.

DESCRIPTION OF THE PRIOR ART

At the present time, certain other unrelated compounds are in experimental clinical use as radiation sensitizers. Said non-related compounds are for example 2-nitro-imidazole derivatives, like the compounds named metronidazol and misonidazole.

Said compounds however suffer from the drawback, that they also cause neurotoxicity which limites their usefulness.

In the European patent publication 0 021 592 there are disclosed compounds having an aminosulfonyl substituent which correspond to the following formula A

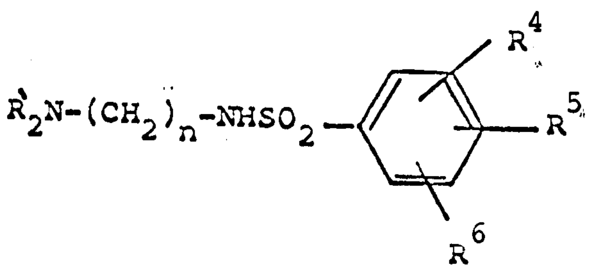

(A)

wherein

the group having the formula

$$NR'_2$$

represents a piperidyl, pyrrolidyl, morpholino or N-methyl piperazyl group, any of which may be substituted by one or two methyl groups and n is 2, 3 or 4.

Accordingly in said European patent publication there are disclosed aminosulfonyl compounds in which to the nitrogen atom of the aminosulfonyl group there is bonded one hydrogen atom and furthermore a straight chain alkyl group comprising 2, 3 or 4 carbon atoms, which is substituted in its omega-position with the nitrogen atom of a heterocyclic nucleus which can be an optionally methyl substituted morpholino group.

In said prior art compounds of formula A the radicals $R^5$ and $R^6$ can be hydrogen or substituents like halo, loweralkyl, hydroxy or alkoxy. Said radicals however can be neither a carboxylic acid group or a corresponding ester or acid amide nor a nitro group.

The radical $R^4$ of said prior art compounds of formula A can be selected from different kinds of substituents including a nitro group as well an esterified carboxylic acid group wherein the alcohol moiety is derived from methanol or ethanol, i.e. the methoxycarbonyl or ethoxycarbonyl group or said group can also

be a carboxamido group. In said European patent publication, however, there are not disclosed corresponding compounds which have in addition to the sulfonamide substituent further a nitro substituent as well as a carboxylic acid ester group or carboxylic acid amide group.

Specifically disclosed are in said European patent publication compounds of formula A wherein an unsubstituted morpholino group is bonded to the nitrogen atom of the sulfonamide group through a straight chain alkyl group comprising 2 or 3 carbon atoms and in which furthermore either

the radical $R^4$ is an acetamido group or a trifluoromethyl group and the radicals $R^5$ and $R^6$ are hydrogen atoms,

or in which each of the radicals $R^4$, $R^5$ and $R^6$ has the meaning of either a chloro atom or a methyl group,

or in which the radical $R^4$ forms together with the radical $R^5$ and the carbon atoms to which they are bonded a cyclohexene structure and the radical $R^6$ is hydrogen. With regard to this reference is made to the corresponding compounds of number 7, 8, 39, 52 and 57 which are disclosed on pages 28, 35, 38 and 43 of said publication.

Accordingly the compounds disclosed in the European patent publication 0 021 592 clearly differ as to their structure from the present compounds and they furthermore have also a completely different pharmaceutical activity, i.e. they are anti-arrhythmic agents.

In the European patent publication 0 197 386 of Merck, which publication however is not prior art but an intermediate publication based on the priority of an older U.S. patent application, i.e. the U.S. patent application 716 886 of March 27, 1985 and the priority of two U.S. patent applications, i.e. the U.S. patent application serial no. 795 569 and 795 564 which both were filed on November 6, 1985, i.e. the same day as the U.S. patent application the priority of which is claimed for the present case.

In said intermediate publication there are described benzoic acids, esters and amides thereof which have a nitro substituent in the position 6 and a sulfonamide substituent in the position 2 to the carboxylic acid group, respectively esters or amides thereof. Said compounds have the following structure B

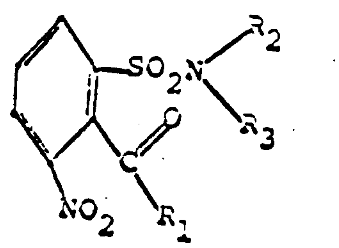

B

wherein

$R_1$ is hydroxy, alkoxy, hydroxyalkoxy, allyloxy, amino, monoalkylamino, dialkylamino, hydroxyalkylamino, di-(hydroxyalkyl)amino or allylamino. If in said prior art compounds of formula B the radicals $R_2$ and $R_3$ which are bonded to the nitrogen atom of the amino sulfonyl group are taken separately they have the meaning of hydrogen, alkyl, hydroxyalkyl, allyl or they are basically substituted alkyl groups selected from the group comprising aminoalkyl, monoalkylaminoalkyl, dialkylaminoalkyl, (hydroxyalkyl)-amino-(loweralkyl), (hydroxyalkyl)-alkylamino-(loweralkyl) and di(hydroxyalkyl)-amino-(loweralkyl).

In said compounds of formula B, however, the radicals

$R_2$ and $R_3$ can also form together with the nitrogen atom to which they are attached a heterocyclic ring radical selected from morpholino or 3-oxo-piperazino-1-yl or $R_4$-substituted 3-oxo-piperazino-1-yl wherein $R_4$ is alkyl or hydroxyalkyl.

The compounds of formula B described in said European patent publication 0 197 386 furthermore have the same pharmaceutical activity as the claimed compounds, i.e. they enhance the therapeutic effect of radiation.

In the European patent publication 0 223 124 which is as well not prior art but an intermediate publication based on the priority of the U.S. patent application serial no. 795 567 of November 6, 1985, which is identical with the priority date claimed for the present case, there are described esters or amides of the 6-nitrobenzoic acid which as well correspond to the above stated formula B if in said formula

$R_1$ is hydroxy-alkoxy having 1 - 4 carbon atoms, alkoxy having 1 - 4 carbon atoms, allyloxy or a group having the formula

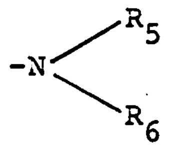

wherein

$R_5$ and $R_6$ are independently from each other hydrogen, alkyl having 1 - 4 carbon atoms, hydroxy-alkyl having 1 - 4 carbon atoms or one of the radicals $R_5$ and $R_6$ is allyl and the other one is hydrogen;

- $R_2$ is hydrogen, alkyl having 1 - 4 carbon atoms, hydroxy-alkyl having 1 - 4 carbon atoms or allyl and
- $R_3$ is an alkyl group which is substituted in its ω-position with a heterocyclic radical, which alkyl group has the following structure

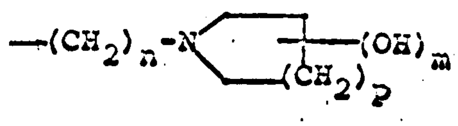

wherein

- n is 2 or 3;
- m is 0, 1 or 2, and
- p is 1 or 2.

The compounds disclosed in said intermediate European patent publication have as well the activity of enhancing the therapeutic effect of radiation.

It was the aim of the present invention to provide further compounds which have the activity of enhancing the therapeutic effect of radiation and which have a structure similar to the structure of the compounds of formula B disclosed in the above stated intermediate European patent publication.

## DETAILED DESCRIPTION OF THE INVENTION

One object of the present invention are substituted 2-aminosulfonyl-6-nitro-benzoic esters or amides of the formula I

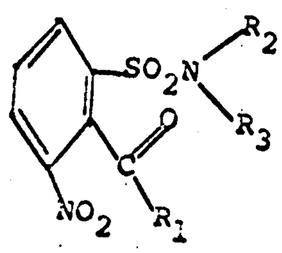 I

wherein

- $R_1$ is alkoxy having 1 - 4 carbon atoms, hydroxyalkoxy having 1 - 4 carbon atoms, allyloxy or a group of formula

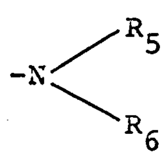

wherein

$R_5$ and $R_6$ are independently from each other hydrogen, alkyl having 1 - 4 carbon atoms or hydroxyalkyl

having 1 - 4 carbon atoms or one of the radicals $R_5$ and $R_6$ is allyl and the other one is hydrogen;

$R_2$ is hydrogen, alkyl having 1 - 4 carbon atoms, hydroxyalkyl having 1 - 4 carbon atoms or allyl and

$R_3$ is a morpholinoalkyl radical of the formula

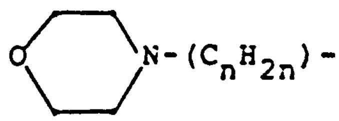

wherein

n is 1, 2 or 3,

or salts of the compounds of formula I.

In preferred inventive compounds of formula I n is 2 or 3, i.e. the morpholino radical is bonded to the nitrogen atom of the sulfonamide group through an ethylene or propylene group.

A further object of the present invention is a process for the preparation of the compounds of formula I or salts of said compounds of formula I, which process is characterized in that the halosulfonyl compound of formula V

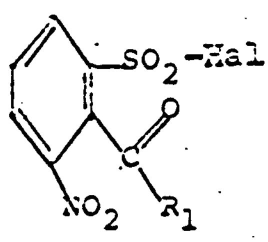

V

wherein Hal is halogen,
is reacted with an amine of formula II

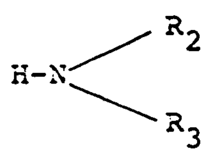

yielding the compounds of formula I which are isolated in the free form or as salts thereof and that optionally compounds of formula I in which $R_1$ is alkoxy having 1 - 4 carbon atoms or hydroxyalkoxy having 1 - 4 carbon atoms are further reacted with ammonia or an amine of formula IV

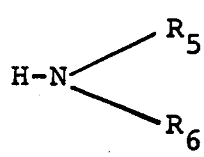

wherein $R_5$ and $R_6$ are as defined in formula I yielding the compounds of formula I in which $R_1$ is a group of formula

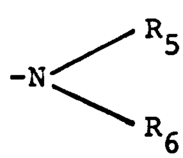

and wherein said acid amides of formula I are isolated in the free form or as salts thereof.

Preferably said process is performed as follows:

The substituted nitrobenzoate ester or nitrobenzamide of formula V which has the 2-halosulfonyl substituent, preferably a corresponding 2-chlorosulfonyl substituent, is reacted with the amine of formula II, which amine accordingly corresponds to the following formula II

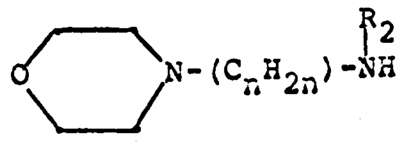

II

wherein

n and $R_2$ are as defined in formula I. Said reaction is preferably performed in an aprotic solvent such as tetrahydrofuran, dioxane, dimethoxyethane, or chloroform and the 2-chlorosulfonyl compound is usually treated with at least an equimolar amount of the amine of formula II.

It is furthermore preferred to carry out the reaction in the presence of a base in sufficient amount to neutralize the hydrogen chloride formed in the course of the reaction. The base utilized may be a tertiary amine such as triethylamine or pyridine. On the other hand the same results may be produced by adding at least twice the molar amount of reactant amine theoretically required. In this event, the reactant amine is utilized both to form the sulfonamide and to neutralize the hydrogen chloride formed in the amination reaction.

The temperature at which the reaction is carried out is not critical and may vary from 0°-100° C or at the reflux temperature of the solvent, if under 130° C. The reaction temperature is preferably maintained at about 0-25° C for a period of 1-24 hours. The amination reaction may be formulated as follows:

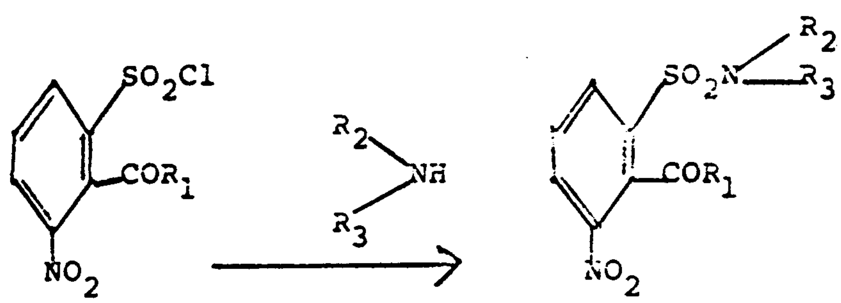

wherein $R_1$, $R_2$ and $R_3$ are as defined hereinabove.

The starting materials for the process are either known or are readily prepared from the known 2-amino-6-nitrobenzoic acid by a process of esterification followed by diazotization of the amino group and treating the formed diazonium compound with $SO_2$ in the presence of $CuCl_2$ whereby the desired starting 2-chlorosulfonyl-6-nitrobenzoate ester is formed.

The benzamide derivatives of this invention may also be prepared by reaction of a 2-(mono-substituted sulfamyl)-6-nitrobenzoate ester of formula III with ammonia or a mono- or dialkyl-substituted amine of formula IV.

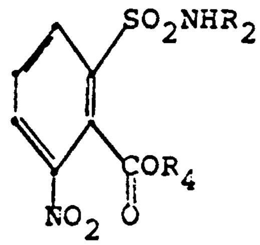

III

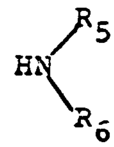

IV

In formula III, $R_2$ is as described hereinabove and $R_4$ is either loweralkyl or hydroxyloweralkyl. In formula IV, $R_5$ and $R_6$ are each separately hydrogen, alkyl, or hydroxyalkyl; or one is allyl and the other one is hydrogen.

Usually the reaction is carried out in a suitable solvent such as a lower aliphatic alcohol or a polar aprotic solvent such as dimethylformamide, dimethylsulfoxide or others such as tetrahydrofuran, glyme, diglyme, chloroform or methylenechloride. The reaction temperature is not critical and may vary from 0-100°C, preferably from about 25°-50°C for a period of 1 to 10 days. When low boiling amines are used, the reaction may be run in a sealed vessel.

A further object of the present invention is a pharmaceutical composition for enhancing the therapeutic effect of radiation, which pharmaceutical composition is characterized in that it contains as pharmaceutically active ingredient a compound of formula I or a pharmaceutically acceptable salt thereof.

Preferably said pharmaceutical compositions contain as further component a non-toxic pharmaceutically acceptable carrier.

The inventive pharmaceutical compositions are intended to be administered to human patients or domestic animals which undergo a radiation treatment of malignant disease processes. The inventive pharmaceutical compositions can be formulated so that they are suitable for an oral or intravenous administration.

The dosage in which said pharmaceutical compositions are employed depends on the radiation protocol for each individual patient. In protocols where the radiation dose is divided into a large number of fractions, the pharmaceutical compositions can be administered at intervals in the schedule and not necessarily with each radiation treatment. It should be noted that the pharmaceutical compositions of the present invention are not intended for chronic administration.

In general the inventive compositions are administered 10 minutes to 5 hours prior to the radiation treatment in such a dosage, that 0,25 grams of the active ingredient of formula I to about 4,0 grams of said active ingredient are administered per square meter of body surface.

The decision as to the exact dosage used, however, must be made by the administering physician based on his judgement of the patient's general physical condition. In determining the dose for the individual patient, the physician may begin with an initial dose of the pharmaceutical composition according to which 0,25 grams of the active ingredient are administered per square meter of body surface in order to determine how well said pharmaceutical composition is tolerated. Thereafter the dosage can be increased with each succeeding radiation treatment, observing the patient carefully for any drug side effect.

The dosage form for intravenous administration is a sterile isotonic solution of the active ingredient of formula I. Oral dosage forms such as tablets, capsules, or elixirs may also be used.

Capsules or tablets containing 25, 50, 100 or 500 mg of the compounds of formula I or a pharmaceutically acceptable salt thereof per capsule or tablet can be formulated and such pharmaceutical preparations are satisfactory for an oral administration.

The following examples are intended to illustrate processes for the preparation of the compounds of formula I. Temperatures are stated in degrees Celsius unless otherwise indicated throughout the application.

EXAMPLE 1

Methyl 2-[N-(2-Morpholinoethyl)aminosulfonyl]-6-nitrobenzoate

A solution of 4-(2-aminoethyl)morpholine (0.95 ml, 7.2 mmol) in THF (25 ml) was added over 25

minutes to a stirred, cooled solution of methyl 2-chlorosulfonyl-6-nitrobenzoate (1.0 g, 3.6 mmol) in THF (100 ml) and the reaction mixture stirred at 20-25°C for 20 hours. After removing TNF under reduced pressure, the residue was partitioned between EtOAc and saturated $Na_2CO_3$ solution. The EtOAc extract was washed (saturated NaCl solution), dried ($Na_2SO_4$), filtered and concentrated. Flash chromatography of the residue over silica gel and elution with 1% MeOH-99% $CHCl_3$ gave 700 mg of pure product. An analytical sample, m.p. 109-11°C, was obtained upon recrystallization from EtOAc-hexane.

EXAMPLE 2

N,N-Dimethyl 2-[N-(2-Morpholinoethyl)aminosulfonyl]-6-nitrobenzamide Hydrochloride

A solution of methyl 2-[N-(2-morpholinoethyl)aminosulfonyl]-6-nitrobenzoate (0.60 g, 1.61 mmol) and 1.8 ml of a 40% aqueous dimethylamine solution in methanol (20 ml) was allowed to stand at 20-25°C for 3 days. After concentrating under reduced pressure, the residue was flash chromatographed over silica gel and eluted with 2% isopropyl alcohol-98% $CH_2Cl_2$ to give 300 mg of product. Treatment with anhydrous ethanolic hydrogen chloride and recrystallization from MeOH-EtOAc-hexane gave the HCl salt, m.p. 206-07°C.

EXAMPLE 3

Methyl 2-[N-(3-Morpholinopropyl)aminosulfonyl]-6-nitrobenzoate

A solution of 4-(3-aminopropyl)morpholine (1.04 g, 7.2 mmol) in THF (25 ml) was added slowly to a cooled solution of methyl 2-chlorosulfonyl-6-nitrobenzoate (1.0 g, 3.6 mmol) in THF (75 ml). After stirring at 20-25°C for 20 hours, THF was removed under reduced pressure and the residue partitioned between EtOAc and a saturated aqueous solution of NaCl. The EtOAc extract was dried ($Na_2SO_4$), filtered and concentrated. Flash chromatography of the residue and elution with 2% MeOH-98% $CHCl_3$ gave 1.4 g of product as an oil.

EXAMPLE 4

N,N-Dimethyl 2-[N-(3-Morpholinopropyl)aminosulfonyl]-6-nitrobenzamide

A solution of methyl 2-[N-(3-morpholinopropyl)aminosulfonyl]-6-nitrobenzoate (1.2 g, 3.1 mmol) and 5 ml of a 40% aqueous dimethylamine solution in MeOH (50 ml) was stirred at 20-25°C for 4 days. After concentrating under reduced pressure, the residue was flash chromatographed over silica gel and eluted with 2% MeOH-98% $CHCl_3$ to give 300 mg of product. An analytical sample, m.p. 104-06°C, was obtained by recrystallization from EtOAc-hexane.

## Claims

**1.** Substituted 2-aminosulfonyl-6-nitro-benzoic esters or amides of the formula I

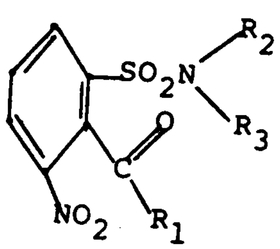

I

wherein

$R_1$ is alkoxy having 1 - 4 carbon atoms, hydroxyalkoxy having 1 - 4 carbon atoms, allyloxy or a group of formula

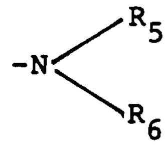

wherein
$R_5$ and $R_6$ are independently from each other hydrogen, alkyl having 1 - 4 carbon atoms or hydroxyalkyl having 1 - 4 carbon atoms or one of the radicals $R_5$ and $R_6$ is allyl and the other one is hydrogen;

$R_2$ is hydrogen, alkyl having 1 - 4 carbon atoms, hydroxyalkyl having 1 - 4 carbon atoms or allyl and

$R_3$ is a morpholinoalkyl radical of the formula

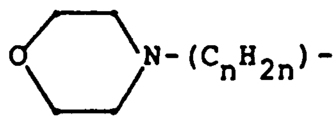

wherein
n is 1, 2 or 3,

or salts of the compounds of formula I.

**2.** Compounds according to claim 1, wherein in formula I in the residue $R_3$ n is 2 or 3 or salts of said compounds.

**3.** Compounds according to claim 1 or 2 which are the following substances:
methyl 2-[N-(2-morpholinoethyl)aminosulfonyl]-6-nitro-benzoate,
N,N dimethyl 2-[N-(2-morpholinoethyl) aminosulfonyl]-6-nitrobenzamide,
methyl 2-[N-(3-morpholinopropyl)aminosulfonyl-6-nitrobenzoate or
N,N-dimethyl-2-[N-(3-morpholinopropyl) aminosulfonyl]-6-nitrobenzamide or
salts of said compounds.

**4.** Process for the preparation of a compound of formula I

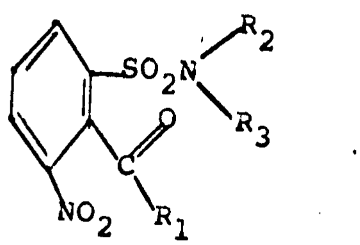

I

according to claim 1 wherein
$R_1$, $R_2$ and $R_3$ have the same meaning as defined in claim 1,
or of salts of said compounds of formula I, characterized in that the halosulfonyl compound of formula V

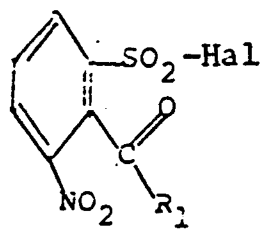

V

wherein Hal is halogen, is reacted with an amine of formula II

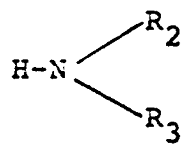

yielding the compounds of formula I which are isolated in the free form or as salts thereof and that optionally compounds of formula I in which $R_1$ is alkoxy having 1 - 4 carbon atoms or hydroxyalxoxy having 1 - 4 carbon atoms are further reacted with ammonia or an amine of formula IV

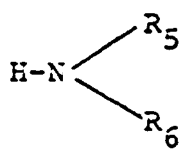

wherein $R_5$ and $R_6$ are as defined in claim 1, yielding the compounds of formula I in which $R_1$ is a group of formula

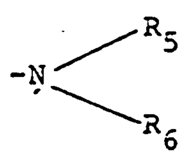

and wherein said acid amides of formula I are isolated in the free form or as salts thereof.

**5.** Process according to claim 4 characterized in that the compounds according to claims 2 or 3 are prepared.

**6.** Pharmaceutical composition for enhancing the therapeutic effect of radiation characterized in that it contains as pharmaceutically active ingredient a compound of formula I according to claim 1 or a pharmaceutically acceptable salt thereof.

**7.** Pharmaceutical composition according to claim 6 characterized in that it contains as pharmaceutically active ingredient a compound according to claim 2 or 3 or a pharmaceutically acceptable salt of said compounds.

**8.** Pharmaceutical composition according to claim 6 or 7 characterized in that it furthermore contains a non-toxic pharmaceutically acceptable carrier.

## Revendications

**1.** Esters ou amides 2-aminosulfonyl-6-nitrobenzoïques substitués de formule I

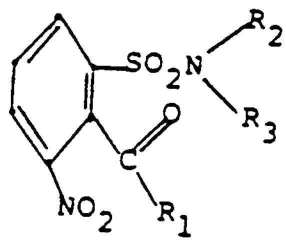

I

dans laquelle

$R_1$ est un groupe alcoxy comportant 1-4 atomes de carbone, hydroxyalcoxy comportant 1-4 atomes de carbone, allyloxy ou un groupe de formule

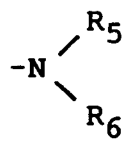

dans laquelle

$R_5$ et $R_6$ sont, indépendamment l'un de l'autre, des atomes d'hydrogène, des groupes alkyle comportant 1-4 atomes de carbone ou hydroxyalkyle comportant 1-4 atomes de carbone, ou l'un des radicaux $R_5$ et $R_6$ est un groupe allyle et l'autre est un atome d'hydrogène;

$R_2$ est un atome d'hydrogène, un groupe alkyle comportant 1-4 atomes de carbone, hydroxyalkyle comportant 1-4 atomes de carbone ou allyle, et

$R_3$ est un radical morpholinoalkyle de formule

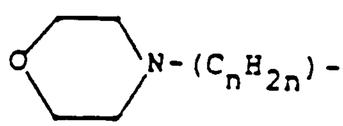

dans laquelle

n est égal à 1, 2 ou 3,

ou sels des composés de formule I.

**2.** Composés selon la revendication 1, dans lesquels, dans la formule I, dans le résidu $R_3$, n est égal à 2 ou 3, ou sels de ces composés.

**3.** Composés selon la revendication 1 ou 2, qui sont les substances suivantes :

2-[N-(2-morpholinoéthyl)aminosulfonyl]-6-nitrobenzoate de méthyle,

N,N-diméthyl-2-(N-(2-morpholinoéthyl)aminosulfonyl]-6-nitrobenzamide,

2-[N-(3-morpholinopropyl)aminosulfonyl]-6-nitrobenzoate de méthyle, ou

N,N-diméthyl-2-[N-(3-morpholinopropyl)aminosulfonyl]-6-nitrobenzamide, ou

sels de ces composés.

**4.** Procédé de préparation d'un composé de formule I

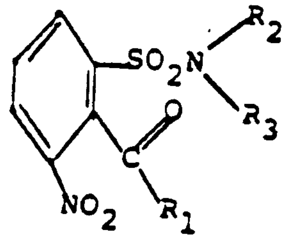

I

selon la revendication 1,
où $R_1$, $R_2$ et $R_3$ ont les mêmes significations que dans la revendication 1,
ou des sels de ces composés de formule I,
caractérisé en ce que l'on fait réagir le composé halogénosulfonyle de formule V

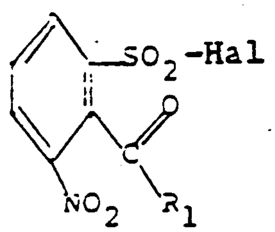

V

dans laquelle Hal est un halogène,
avec une amine de formule II

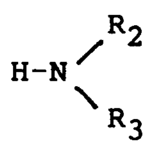

pour obtenir les composés de formule I que l'on isole sous forme libre ou sous forme de leurs sels,
et en ce que l'on fait en outre réagir, éventuellement, les composés de formule I dans lesquels $R_1$ est un groupe alcoxy comportant 1-4 atomes de carbone ou hydroxyalcoxy comportant 1-4 atomes de carbone, avec de l'ammoniac ou une amine de formule IV

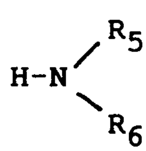

dans laquelle $R_5$ et $R_6$ sont tels que définis dans la revendication 1, pour obtenir les composés de formule I, dans lesquels $R_1$ est un groupe de formule

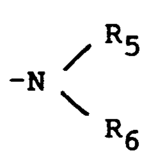

et on isole les amides d'acide de formule I sous forme libre ou sous forme de leurs sels.

5. Procédé selon la revendication 4, caractérisé en ce que l'on prépare les composés selon les revendications 2 ou 3.

6. Composition pharmaceutique pour améliorer l'effet thérapeutique d'un rayonnement, caractérisée en ce qu'elle contient, comme ingrédient pharmaceutiquement actif, un composé de formule I selon la revendication 1 ou un de ses sels pharmaceutiquement acceptables.

7. Composition pharmaceutique selon la revendication 6, caractérisée en ce qu'elle contient, comme ingrédient pharmaceutiquement actif, un composé selon la revendication 2 ou 3 ou un de ses sels pharmaceutiquement acceptables.

8. Composition pharmaceutique selon la revendication 6 ou 7, caractérisée en ce qu'elle contient, en outre, un véhicule non toxique pharmaceutiquement acceptable.

**Patentansprüche**

1. Substituierte 2-Aminosulfonyl-6-nitrobenzoesäureester oder -amide der Formel I

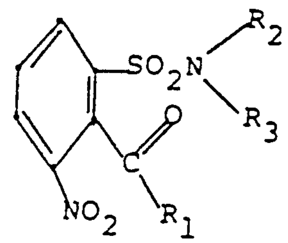

I

worin

$R_1$ Alkoxy mit 1 bis 4 Kohlenstoffatomen, Hydroxyalkoxy mit 1 bis 4 Kohlenstoffatomen, Allyloxy oder eine Gruppe der Formel

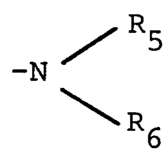

ist, worin

$R_5$ und $R_6$ unabhängig voneinander Wasserstoff, Alkyl mit 1 bis 4 Kohlenstoffatomen oder Hydroxyalkyl mit 1 bis 4 Kohlenstoffatomen sind oder einer der Reste $R_5$ und $R_6$ Allyl ist und der andere Wasserstoff ist;

$R_2$ Wasserstoff, Alkyl mit 1 bis 4 Kohlenstoffatomen, Hydroxyalkyl mit 1 bis 4 Kohlenstoffatomen oder Allyl ist und

$R_3$ ein Morpholinoalxylrest der Formel

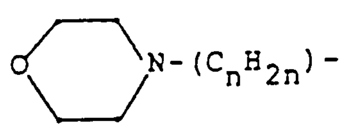

ist, worin

n 1, 2 oder 3 ist,

oder Salze der Verbindungen der Formel I.

**2.** Verbindungen nach Anspruch 1, worin in Formel I im Rest $R_3$ n 2 oder 3 ist, oder Salze dieser Verbindungen.

**3.** Verbindungen nach Anspruch 1 oder 2, welche die folgenden Substanzen sind:
Methyl-2-[N-(2-morpholinoethyl)aminosulfonyl]-6-nitrobenzoat,
N,N-Dimethyl-2-[N-(2-morpholinoethyl)aminosulfonyl]-6-nitrobenzamid,
Methyl-2-[N-(3-morpholinopropyl)aminosulfonyl-6-nitrobenzoat oder
N,N-Dimethyl-2-[N-(3-morpholinopropyl)aminosulfonyl]-6-nitrobenzamid oder
Salze dieser Verbindungen sind.

**4.** Verfahren zur Herstellung einer Verbindung der Formel I

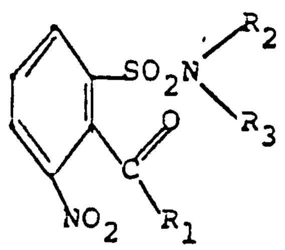

I

nach Anspruch 1, worin $R_1$, $R_2$ und $R_3$ die gleiche Bedeutung haben, wie in Anspruch 1 definiert, oder von Salzen der Verbindungen der Formel I, dadurch gekennzeichnet, daß die Halogensulfonylverbindung der Formel V

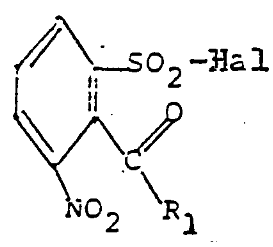

V

worin Hal Halogen ist,
mit einem Amin der Formel II umgesetzt wird

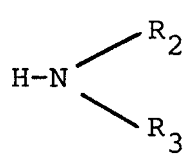

wobei die Verbindungen der Formel I erhalten werden, die in freier Form oder als Salze davon isoliert werden, und daß gegebenenfalls Verbindungen der Formel I, worin $R_1$ Alkoxy mit 1 bis 4 Kohlenstoffatomen oder Hydroxyalkoxy mit 1 bis 4 Kohlenstoffatomen ist, weiter mit Ammoniak oder einem Amin der Formel IV

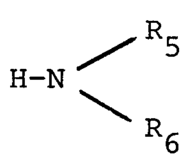

worin $R_5$ und $R_6$ wie in Anspruch 1 definiert sind, unter Erhalt der Verbindungen der Formel I, worin $R_1$

eine Gruppe der Formel

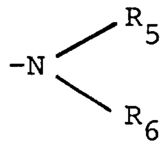

ist und worin die Säureamide der Formel I in der freien Form oder als Salz davon isoliert werden, umgesetzt werden.

5. Verfahren nach Anspruch 4, dadurch gekennzeichnet, daß die Verbindungen nach Anspruch 2 oder 3 hergestellt werden.

6. Pharmazeutische Zusammensetzung zur Verstärkung der therapeutischen Wirkung von Strahlung, dadurch gekennzeichnet, daß sie als pharmazeutisch wirksamen Bestandteil eine Verbindung der Formel I nach Anspruch 1 oder ein pharmazeutisch annehmbares Salz davon enthält.

7. Pharmazeutische Zusammensetzung nach Anspruch 6, dadurch gekennzeichnet, daß sie als pharmazeutisch wirksamen Bestandteil eine Verbindung nach Anspruch 2 oder 3 oder ein pharmazeutisch annehmbares Salz dieser Verbindungen enthält.

8. Pharmazeutische Zusammensetzung nach Anspruch 6 oder 7, dadurch gekennzeichnet, daß sie weiterhin einen nicht toxischen pharmazeutisch annehmbaren Träger enthält.